# EUROPÄISCHE PATENTSCHRIFT

(11) **EP 2 039 290 B1**
(45) Veröffentlichungstag und Bekanntmachung des Hinweises auf die Patenterteilung: **21.04.2010**
(21) Anmeldenummer: 08014974.3
(22) Anmeldetag: 22.08.2008
(51) Int. Cl.: A61B 5/02

(54) **Vorrichtung zur Messung von medizinischen Parametern des menschlichen Körpers**
Device for measuring medical parameters of the human body
Dispositif de mesure de paramètres médicaux du corps humain

(30) Priorität: 20.09.2007 DE 102007045151
(43) Veröffentlichungstag der Anmeldung: 25.03.2009
(73) Patentinhaber: Medisana AG, 40724 Hilden (DE)
(72) Erfinder: Lindner, Ralf, 40597 Düsseldorf (DE)
(74) Vertreter: Ostriga, Sonnet, Wirths & Roche

(56) Entgegenhaltungen:
- EP-A- 1 714 611
- DE-U1-202004 005 764
- US-A- 5 485 848

## Beschreibung

Die Erfindung betrifft eine Vorrichtung zur Messung von medizinischen Parametern des menschlichen Körpers, insbesondere Blutdruck und Puls, im wesentlichen bestehend aus einer aufpumpbaren Oberarmmanschette, welche über einen Manschettenschlauch mit eine Gehäuse einer Bedienungs-, Anzeige- und Antriebseinrichtung verbunden ist.

Ein derartiges Blutdruckmessgerät ist allgemein aus dem druckschriftlich nicht belegbaren Stand der Technik bekannt. Im Zusammenhang damit, dass chronisch kreislaufkranke Personen meist derartige Vorrichtungen ständig, auch während Reisen, bei sich führen müssen, wird jedoch bei der Vorrichtung nach dem Stand der Technik die Baugröße als verbesserungswürdig angesehen.

Die Aufgabe der Erfindung besteht deshalb darin, eine neue Vorrichtung zur Messung von medizinischen Parametern des menschlichen Körpers, insbesondere Blutdruck und Puls, zu schaffen, welche - insbesondere im Reisegepäck - weniger Platz beansprucht und einfach zu bedienen ist.

Die Lösung der Aufgabe ergibt sich aus den Merkmalen des Patentanspruchs 1, insbesondere dem Kennzeichenteil, wonach das Gehäuse eine schwenkbare Bedienungs- und Anzeigeeinrichtung aufweist, welches in Abhängigkeit von der Winkelstellung zur Horizontalen als Bedienungs- und Anzeigeeinrichtung zum Messen von Blutdruck/Puls bzw. als Reisewecker mit Uhr fungiert.

Der wesentliche Vorteil der Erfindung besteht zunächst darin, dass die erfindungsgemäße Vorrichtung eine Mehrfachfunktion aufweist und insbesondere auf Reisen den separaten Transport von Blutdruckmessgerät und Reisewecker nebeneinander vermeidet. Auch ist die erfindungsgemäße Vorrichtung einfach aufgebaut und kann lediglich durch Verschwenken der Bedienungs- und Anzeigeeinrichtung in die verschiedenen Funktionsstellungen gebracht werden.

Bei einer vorteilhaften Ausführungsform ist die Bedienungs- und Anzeigeeinrichtung im Bereich bis ca. 60° zum Messen von Blutdruck und Puls und im Bereich von ca. 60 - 90° als Reisewecker mit Funkuhr verwendbar. In diesem Zusammenhang ist es auch möglich, dass die Umschaltung der Bedienungs- und Anzeigeeinrichtung von einem Bereich in den anderen Bereich automatisch lediglich durch Verschwenken der Einrichtung erfolgt, wodurch der Bedienungskomfort vergrößert wird.

Bei einer vorteilhaften Ausführungsform ist darüber hinaus eine automatische Sperrung der Bedienungs- und Anzeigeeinrichtung in der horizontalen Anordnung vorhanden.

Letztlich weist eine besonders bevorzugte Ausführungsform der Erfindung ein Gehäuse auf, welches aus einem unteren und einem oberen, die Bedienungs- und Anzeigeeinrichtung aufweisenden Gehäuseteil gebildet wird, welche miteinander im Bereich einer gemeinsamen Schwenkachse verbunden sind, wobei zwischen den Gehäuseteilen ein beidseitig offener Hohlraum zur Aufnahme der Oberarmmanschette einschließlich Manschettenschlauch vorhanden ist. Diese Ausführungsform zeichnet sich durch eine modulare sehr einfach herzustellende und zu montierende Bauweise aus, wobei diese Ausführungsform zusätzlich sehr kompakt ausgebildet ist, da sie auch noch einen Aufnahmeraum für die Oberarmmanschette aufweist.

Weitere Vorteile der Erfindung ergeben sich aus dem nachfolgenden Unteranspruch sowie aus der Beschreibung eines Ausführungsbeispieles. Es zeigen:
Fig. 1 eine perspektivische Ansicht einer Vorrichtung zur Messung von medizinischen Parametern des menschlichen Körpers,
Fig. 2 eine Darstellung der Vorrichtung gemäß Fig. 1 in einer anderen Stellung der Bedienungs- und Anzeigeeinrichtung,
Fig. 3 eine isolierte Darstellung der Oberarmmanschette einschließlich Schlauch,
Fig. 4 - 6 Seitendarstellung einer Vorrichtung gemäß Fig. 1 in geschlossener teilgeöffneter bzw. vollständig geöffneter Stellung,
Fig. 7 eine Explosionsdarstellung der Vorrichtung gemäß Fig. 1 und
Fig. 8 eine schematische Draufsicht auf die Vorrichtung gemäß Fig. 1 ohne oberes Gehäuseteil.

In den Zeichnungen ist insgesamt eine Vorrichtung 10 zur Messung von medizinischen Parametern des menschlichen Körpers, insbesondere Blutdruck und Puls, dargestellt.

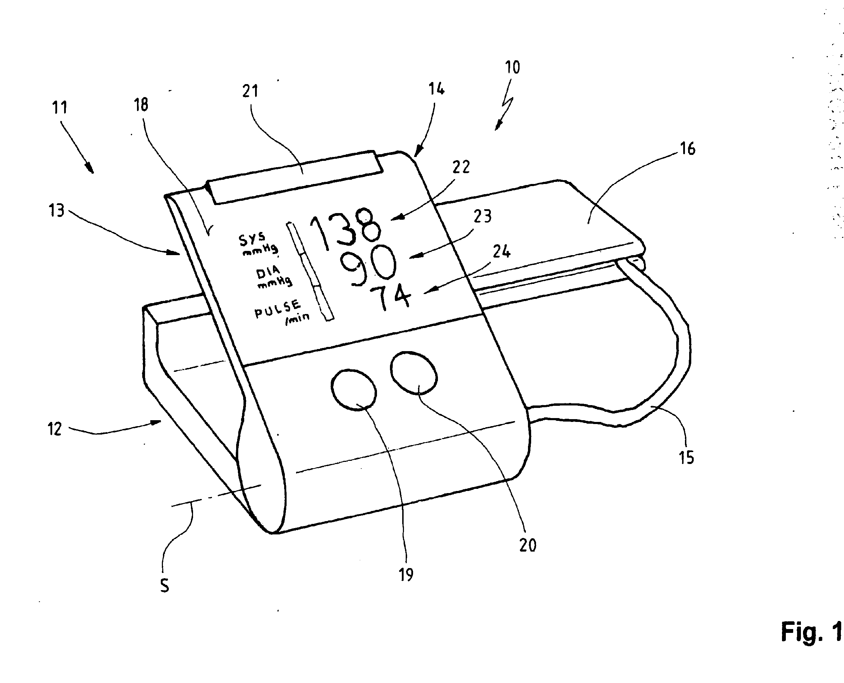

Die Fig. 1 zeigt in perspektivischer Ansicht eine derartige Vorrichtung 10. Man erkennt das Gehäuse 11, welches aus einem Unterteil 12 und einem dazu verschwenkbaren Oberteil 13 gebildet wird. Auf der Oberseite 13 ist eine Bedienungs- und Anzeigeeinrichtung 14 angeordnet. Im Bereich einer Schwenkachse S - auf die nachfolgend noch eingegangen wird - ist ein Manschettenschlauch 15 angeordnet, der das Gehäuse 11 mit einer aufpumpbaren Oberarmmanschette 16 verbindet.

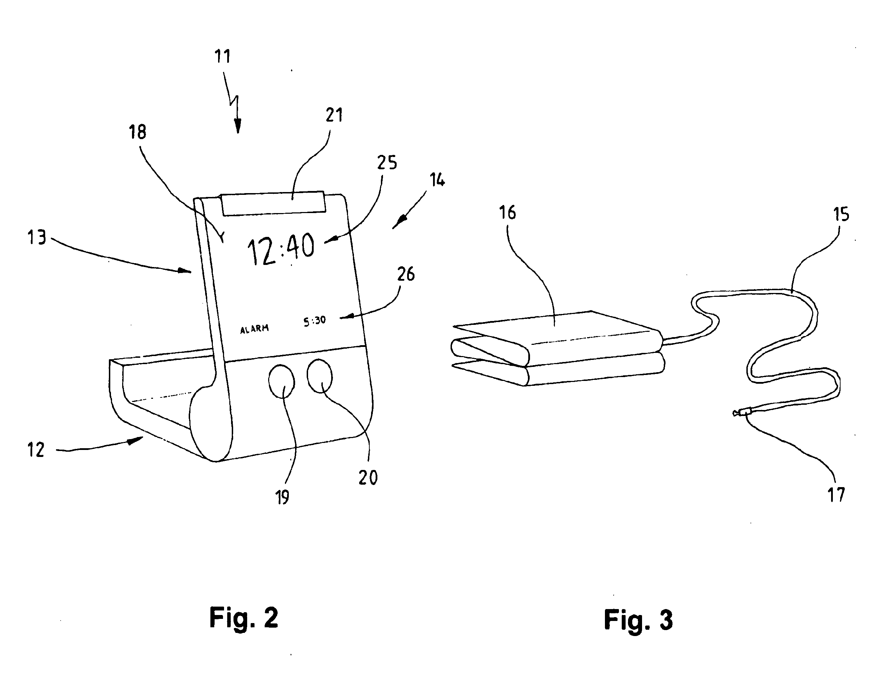

In den Figuren 2 und 3 ist wiederum die Vorrichtung 10, jedoch getrennt in der Fig. 2 das Gehäuse 11 und in Fig. 3 die Oberarmmanschette 16 einschließlich Manschettenschlauch 15 sowie ein Kupplungsteil 17 zu erkennen.

In Verbindung mit den Zeichnungen 4, 5 und 6 erkennt man die wesentlichen funktionellen Stellungen der Vorrichtung 10.

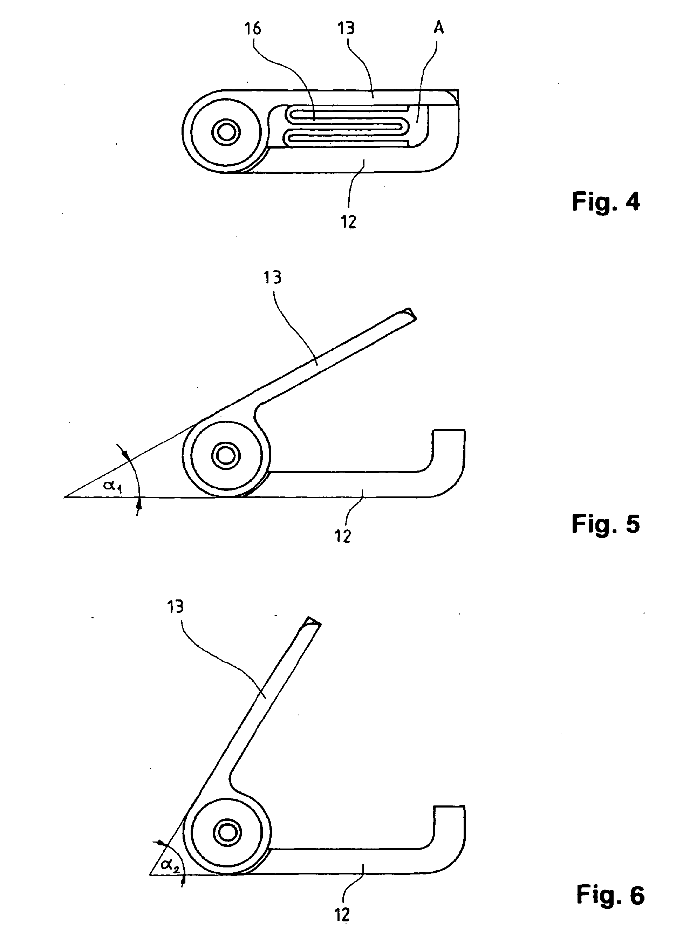

In der Fig. 4 ist die Vorrichtung 10 mit geschlossenem Gehäuse 11 dargestellt. Man sieht, dass das Oberteil 13 vollständig am Unterteil 12 anliegt, und dass somit zwischen dem Unterteil 12 und dem Oberteil 13 ein Winkel von 0° vorhanden ist. Zwischen dem Unterteil 12 und dem Oberteil 13 ist ein Aufnahmeraum A angeordnet, in dem die Oberarmmanschette 16 und auf nicht dargestellte Weise auch der Manschettenschlauch 15 einschließlich Kupplungsstecker 17 verstaut sind.

In der Fig. 5 ist in Seitenansicht die Vorrichtung 10 dargestellt, wobei das Unterteil 12 in einem Winkel von α₁ < 60° zum Oberteil 13 angeordnet ist. In dieser Stellung arbeitet die Vorrichtung 10 automatisch als Blutdruckmessgerät. Hierzu ist insbesondere auch auf die Fig. 1 zu verweisen. Dort erkennt man zunächst einmal, dass die Bedienungs- und Anzeigeeinrichtung 14 ein Anzeigedisplay 18 sowie Betätigungseinrichtungen 19, 20 und 21 aufweist.

Sollte bei der in den Figuren 1 und 5 gezeigten Stellung die Oberarmmanschette 16 am Oberarm eines nicht dargestellten Menschen befestigt sein, so ist auf dem Anzeigedisplay 18, wie in Fig. 1 dargestellt, beispielsweise mit 22 ein systolischer Druck von 138 mm Quecksilbersäule, mit 23 ein diastolischer Druck von 90 mm Quecksilbersäule sowie mit 24 ein Puls von 74 angezeigt.

Aus der Fig. 2 in Verbindung mit der Fig. 6 erkennt man einen weiteren Arbeitsbereich der Vorrichtung 10, in dem diese als Reisewecker mit Uhr, vorzugsweise Funkuhr, fungiert. Man erreicht diesen Arbeitsbereich automatisch durch ein weiteres Verschwenken des Oberteils 13 gegenüber dem Unterteil 12 über die Grenze von α₂ = 60° hinweg bis maximal 90°. Sofern die Vorrichtung 10 als Reisewecker mit Uhr fungiert, ändert sich - wie in Fig. 2 dargestellt - automatisch das Anzeigedisplay 18 und es erscheint neben einer aktuellen Uhrzeit 25 auch eine eingegebene Alarmzeit 26. Auch erhalten die Betätigungseinrichtungen 19, 20 und 21 eine andere Funktion.

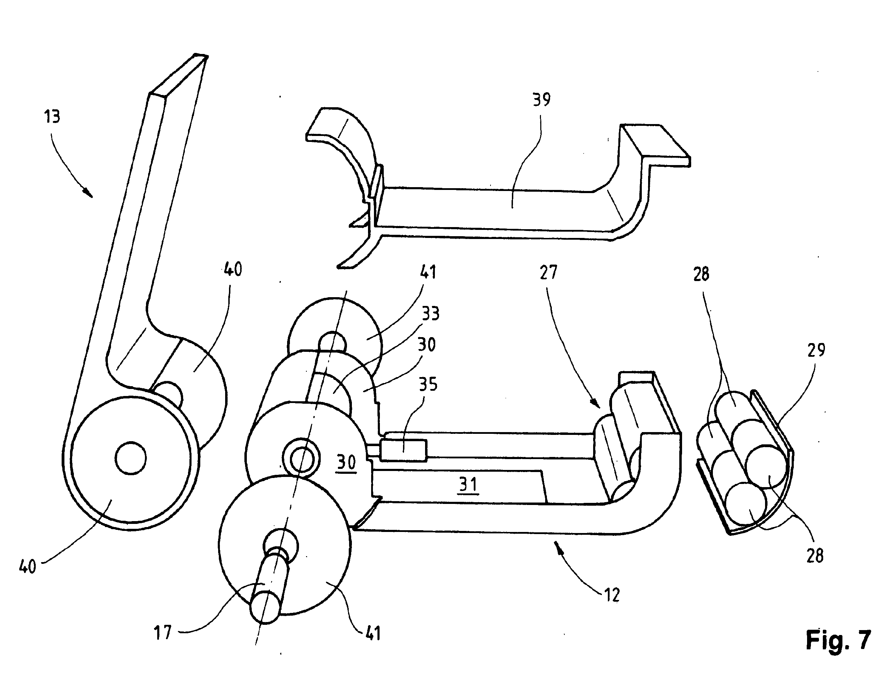

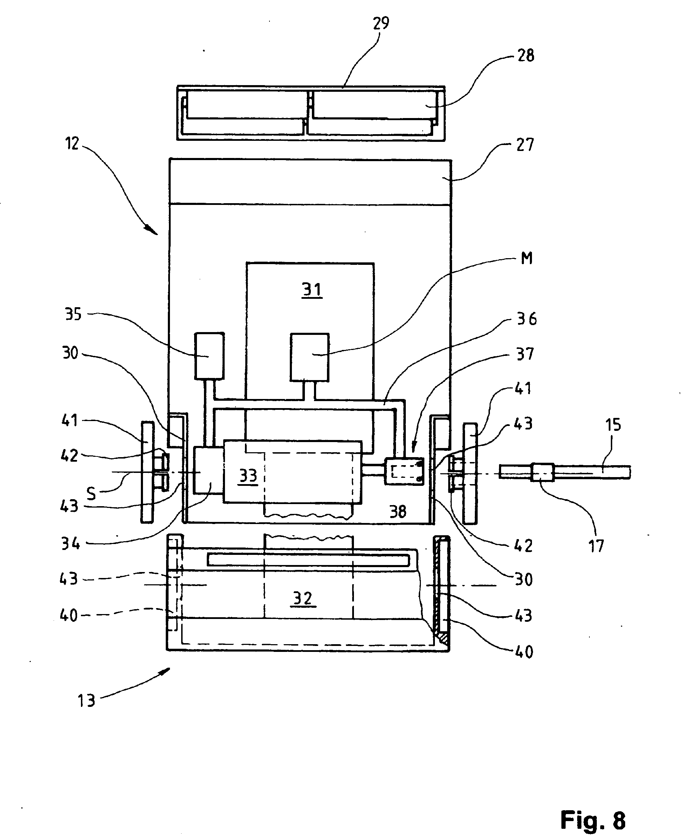

Aus den Figuren 7 und 8 ergibt sich im Einzelnen der Aufbau der Vorrichtung 10 zur Messung von medizinischen Parametern des menschlichen Körpers.

In der Fig. 7 sind die wesentlichen Teile der Vorrichtung 10 in Explosionsdarstellung zu erkennen. Zunächst einmal ist das Unterteil 12 mit seinem Batteriefach 27 sowie den zugehörigen Batterien 28 einschließlich Verschlussdeckel 29 zu erkennen. Gegenüberliegend ist das Unterteil 12 beidseitig mit Achsaufnahmen 30 versehen. Aus der Fig. 7 in Verbindung mit Fig. 8 erkennt man des Weiteren, dass im mittleren Bereich des Unterteils 12 eine Steuerungsplatine 31 angeordnet ist, welche über eine flexible, gedruckte Leiterbahn 32 mit der Bedienungs- und Anzeigeeinrichtung 14 im Oberteil 13 verbunden ist.

Im Bereich der Schwenkachse S ist im Unterteil 12 eine Pumpvorrichtung 33 nebst Schnellentlüftungsventil 34 sowie einem Entlüftungsventil 35 vorgesehen. Das Schnellentlüftungsventil 34 und Entlüftungsventil 35 sind über eine Verbindungsleitung 36 mit einer im Gehäuse 11 angeordneten Kupplungsaufnahme 37 für das Kupplungsteil 17 des Manschettenschlauchs 15 versehen. Die Kupplungsaufnahme 37 ist wiederum über die Verbindungsleitung 38 mit der Pumpeinrichtung 33 verbunden. Im Bereich der Steuerungsplatine 31 ist ein Messsensor M angeordnet, der an die Verbindungsleitung 36 angeschlossen ist. Er dient zur Erfassung des Druckes in dem Manschettenschlauch 15 bei der Oberarmmanschette 16.

Die im Unterteil 12 angeordneten, zuvor beschriebenen Einrichtungen werden im montierten Zustand von einer Abdeckung 39 geschützt. Wie aus der Fig. 7 aber auch aus der Fig. 8 in teilweiser Explosionsdarstellung erkennbar, weist auch das Oberteil 13 Achsaufnahmen 40 auf, welche letztendlich der Verbindung von Unter- und Oberteil 12 und 13 dienen. Hierzu wird, wie insbesondere Fig. 8 zeigt, das Oberteil 13 mit seiner Achsaufnahme 40 so im Bereich der Achsaufnahmen 30 des Unterteiles 12 positioniert, dass Achskappen 41, welche innenseitig beabstandete Rastmittel 42 aufweisen, durch miteinander fluchtende Bohrung 43 gedrückt werden können, so dass eine feste schwenkbare Rastverbindung zwischen Unter- und Oberteil 12, 13 entsteht. Da die der Kupplungsaufnahme 37 gegenüberliegende Achskappe 41 eine Bohrung 44 aufweist, die ebenfalls mit der Bohrung 43 fluchtet, besteht die Möglichkeit, das Kupplungsteil 17 des Manschettenschlauchs 15 in der Kupplungsaufnahme 37 zu verankern. Damit ist eine durchgehende, lösbare Verbindung von der Pumpvorrichtung 33 bis zur Oberarmmanschette 16 vorhanden.

Für den Fall, dass die Vorrichtung 10 als Blutdruckmessgerät im Einsatz ist, kann durch Betätigung der Bedienungseinrichtungen 19, 20 oder 21 die Pumpeinrichtung 33 inganggesetzt werden, so dass über die Verbindungsleitung 38, die Kupplungsaufnahme 37 und den Manschettenschlauch 15 die Oberarmmanschette 16 mit Luft gefüllt wird. Das Ablassen der Luft kann entweder schlagartig über das Schnellentlüftungsventil 34 oder stufenweise über das Entlüftungsventil 35, z. B. nach vorheriger Betätigung einer der Betätigungseinrichtungen 19, 20 oder 21 erfolgen.

## Patentansprüche

1. Vorrichtung zur Messung von medizinischen Parametern des menschlichen Körpers, insbesondere Blutdruck und Puls, im wesentlichen bestehend aus einer aufpumpbaren Oberarmmanschette, welche über einen Manschettenschlauch mit einem Gehäuse einer Bedienungs-, Anzeige- und Antriebseinrichtung verbunden ist, **dadurch gekennzeichnet, dass** das Gehäuse (11) eine schwenkbare Bedienungs- und Anzeigeeinrichtung (14) aufweist, welches in Abhängigkeit von der Winkelstellung (α₁, α₂) zur Horizontalen als Bedienungs- und Anzeigeeinrichtung (14) zum Messen von Blutdruck/Puls bzw. als Reisewecker mit Uhr fungiert.

2. Vorrichtung nach Anspruch 1, **dadurch gekennzeichnet, dass** die Bedienungs- und Anzeigeeinrichtung (14) im Bereich bis ca. 60° zum Messen von Blutdruck und Puls und im Bereich von ca. 60 bis 90° als Reisewecker mit Uhr verwendbar ist.

3. Vorrichtung nach Anspruch 1 oder 2, **dadurch gekennzeichnet, dass** die Bedienungs- und Anzeigeeinrichtung (14) in der Horizontalanordnung gesperrt ist.

4. Vorrichtung nach Anspruch 2 oder 3, **dadurch gekennzeichnet, dass** die Umschaltung der Bedienungs- und Anzeigeeinrichtung (14) von einem Bereich in den anderen Bereich automatisch durch Verschwenken der Einrichtung (14) erfolgt.

5. Vorrichtung nach einem der Ansprüche 1 bis 3, **dadurch gekennzeichnet, dass** das Gehäuse (11) aus einem unteren und einem oberen, die Bedienungs- und Anzeigeeinrichtung (14) aufweisenden Gehäuseteil (12, 13) gebildet wird, welche miteinander im Bereich einer gemeinsamen Schwenkachse (S) verbunden sind und das zwischen den Gehäuseteilen (12, 13) ein beidseitig offener Aufnahmeraum (A) zur Aufnahme der Oberarmmanschette (16) einschließlich Manschettenschlauch (15) vorhanden ist.

6. Vorrichtung nach einem der vorangehenden Ansprüche, **dadurch gekennzeichnet, dass** die die Gehäuseteile (12, 13) verbindende Schwenkanordnung teilweise hohl ausgebildet ist und der lösbaren Verbindung von im Gehäuse (11) angeordneter Pumpeinrichtung (33) mit dem Manschettenschlauch (15) dient.

## Claims

1. Device for measuring medical parameters of the human body, in particular blood pressure and pulse, said device essentially consisting of an upper-arm sleeve which can be pumped up and which is connected, via a hose of said sleeve, to a casing of an operating, display and energising apparatus,
**characterised in that** the casing (11) has a pivoting operating and display apparatus (14) which, depending upon the angular position (α₁, α₂) in relation to the horizontal, functions as an operating and display apparatus (14) for measuring blood pressure/pulse or as a travelling alarm with a clock.

2. Device according to claim 1, **characterised in that** the operating and display apparatus (14) can be used, within the range up to about 60°, for measuring blood pressure and pulse and, within the range from about 60 to 90°, as a travelling alarm with a clock.

3. Device according to claim 1 or 2, **characterised in that** the operating and display apparatus (14) is arrested in the horizontal disposition.

4. Device according to claim 2 or 3, **characterised in that** the changing-over of the operating and display apparatus (14) from one range to the other takes place automatically as a result of the tilting of said apparatus (14).

5. Device according to one of claims 1 to 3,
**characterised in that** the casing (11) is formed from a lower part (12) and an upper part (13) which comprises the operating and display apparatus (14), which parts are connected to one another in the region of a common axis of pivoting (S), and that, between the parts (12, 13) of the casing, there is a receiving compartment (A), which is open on both sides, for receiving the upper-arm sleeve (16) including the hose (15) of said sleeve.

6. Device according to one of the preceding claims,
**characterised in that** the pivoting arrangement that connects the parts (12, 13) of the casing is partly of hollow construction and serves for detachably connecting a pumping apparatus (33) disposed in the casing (11) to the hose (15) of the sleeve.

## Revendications

1. Dispositif de mesure de paramètres médicaux du corps humain, en particulier de la tension sanguine et du pouls, composé essentiellement d'une manchette de partie supérieure du bras, susceptible d'être gonflée par pompage, reliée, par un tuyau souple de manchette, à un boîtier d'un dispositif de manoeuvre, d'affichage et d'entraînement, **caractérisé en ce que** le boîtier (11) présente un dispositif de manoeuvre et d'affichage (14) susceptible de pivoter, servant à la mesure de la pression sanguine/du pouls, ou de réveil de voyage muni d'une horloge, en fonction de la position angulaire (α₁, α₂) par rapport à l'horizontale.

2. Dispositif selon la revendication 1, **caractérisé en ce que** le dispositif de manoeuvre et d'affichage (14) est utilisable pour la mesure de la pression sanguine/du pouls dans la plage angulaire allant jusqu'à à peu près 60° et servant de réveil de voyage muni d'une horloge dans la plage angulaire allant d'à peu près 60 à 90°.

3. Dispositif selon la revendication 1 ou 2, **caractérisé en ce que** le dispositif de manoeuvre et d'affichage (14) est bloqué lorsqu'il se trouve en position horizontale.

4. Dispositif selon la revendication 2 ou 3, **caractérisé en ce que** la commutation du dispositif de manoeuvre et d'affichage (14) s'effectue d'une plage dans l'autre, automatiquement, par pivotement du dispositif (14).

5. Dispositif selon l'une des revendications 1 à 3, **caractérisé en ce que** le boîtier (11) est formé d'une partie de boîtier inférieure et d'une supérieure (12, 13), présentant le dispositif de manoeuvre et d'affichage (14), reliées ensemble dans la zone d'un axe de pivotement (S) commun et **en ce que**, entre les parties (12, 13)de boîtier, est prévu un espace de réception (A) ouvert de part et d'autre, pour recevoir la manchette de partie supérieure du bras (16), y compris le tuyau souple de manchette (15).

6. Dispositif selon l'une des revendications précédentes, **caractérisé en ce que** l'agencement pivotant, reliant les parties (12, 13) de boîtier, est partiellement creux et sert à la liaison désolidarisable du dispositif à pompe (33), disposé dans le boîtier (11), au tuyau souple de manchette (15).
